# EUROPEAN PATENT APPLICATION

(11) **EP 2 238 998 A1**
(43) Date of publication of application: **13.10.2010**
(21) Application number: 09157146.3
(22) Date of filing: 02.04.2009
(51) Int. Cl.: A61M 5/162

(54) **Cannula for piercing a septum of a cartridge and valve for the cannula**

(71) Applicant: F. Hoffmann-La Roche AG, 4070 Basel (CH); Roche Diagnostics GmbH, 68305 Mannheim (DE)
(72) Inventor: Niklaus, Hanspeter, 4853, Riken (CH); Haenggi, Roger, 4208, Nunningen (CH); Johner, Pascal, 3286, Muntelier (CH)
(74) Representative: Schwabe - Sandmair - Marx

(57) **Abstract**

The inventions describes a cannula for piercing a septum (7) of a vial or cartridge (8) comprising a penetrating element (1), at least one dispensing opening (6) of the cannula being in fluid connection with at least one intake opening (4a, 4b, 4c), wherein the penetrating element (1) comprises at least one cutting edge (1a, 1b, 1c) extending in a longitudinal direction of the penetrating element (1) to at least partially overlap in the longitudinal direction with the at least one intake opening (4a, 4b, 4c).

## Description

The invention relates to a cannula, in particular for piercing a septum of a vial or cartridge such as a drug cartridge for an insulin pump. The cannula may be part of a disposable adaptor connecting a drug cartridge and an infusion tubing. The cannula may be used for puncturing a preferably elastic material, such as the septum of a cartridge which is made for example of rubber, plastics or other materials.

EP 0 452 595 A1 discloses a catheter assembly which comprises a tubing comprising a body portion and a hollow needle portion associated therewith, at least one of said body portion and said needle portion being fabricated of a liquid crystalline polymer.

US 5,454,409 discloses a transfer adaptor for use with a vial, the adaptor being made of plastic.

EP 0 174 011 A2 discloses a medical puncture needle moulded with a mixture of thermoplastic resin and a fibrous material dispersed in the resin.

US 2,746,455 discloses a fluid-conveying piercing needle.

US 3,119,391 discloses a needle-like piercing device for puncturing a variety of materials for use in the field of parenteral fluid administration.

US 4,058,121 discloses a thermoplastic needle for injecting sterile liquid into a vial with lateral turbulent motion while air from such vial escapes through a special vent groove on the needle.

US 4,020,837, which corresponds to DE 2 649 881 A1, discloses a hollow piercing tip for vial stoppers.

US 4,838,877 discloses a polymeric hypodermic device.

US 2,954,768 discloses a puncture point useful for connecting administration sets to containers of blood or solutions for parenteral and tubal use.

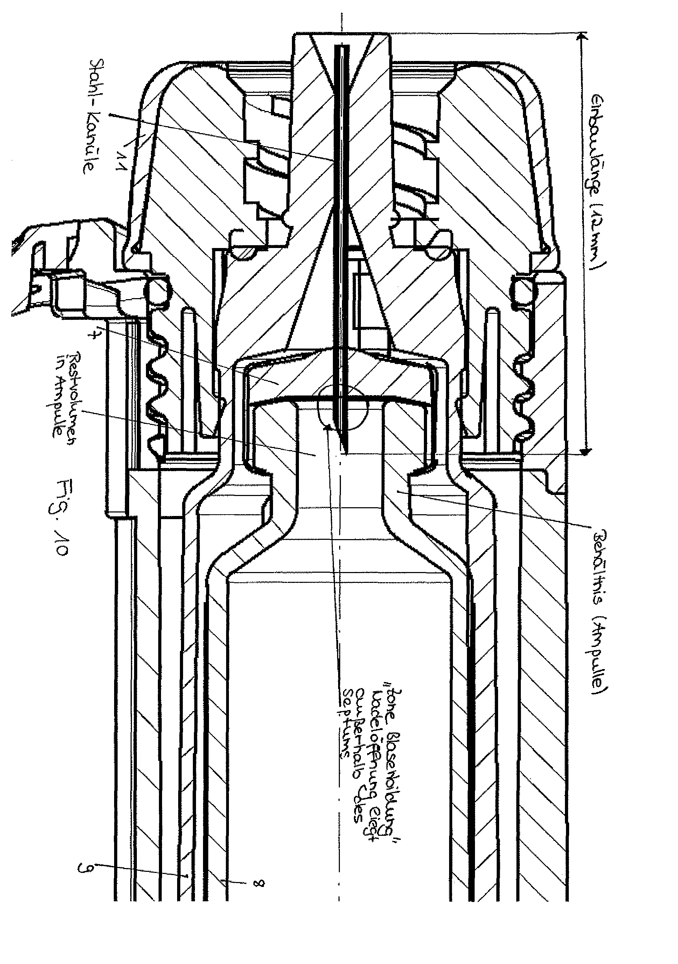

The use of a sterile steel cannula to penetrate the septum of a cartridge, as shown in Figure 10 is already known. However, nickel is often contained in such a steel cannula, which can lead to an allergic reaction on the part of the user. Moreover, it is cumbersome to produce an adaptor using such a steel cannula, which has to be safely fitted and also correctly aligned within the surrounding plastics. The steel cannula is designed to have a length which guarantees full penetration of the septum when inserted into a cartridge, such that the opening of the steel cannula is spaced apart from the inner side of the septum when fully inserted. However, this can lead to problems when trying to discharge air from the cannula (priming).

It is an object of the present invention to provide a cannula which can be used to pierce an elastic material such as a septum or to access the inside of a vial or cartridge.

A cannula for piercing a septum or a sealing element, or for accessing the inside of a vial or cartridge containing for example a substance to be dispensed, such as for example insulin or a growth hormone, comprises a penetrating element having a tip or point and/or at least one cutting edge and preferably two, three or more cutting edges disposed around or on the penetrating element and preferably each extending substantially in a longitudinal direction. The penetrating element has a longitudinal direction or axis which can be defined as an axis running from the tip or piercing point to the opposite end, or from the inside of a vial or cartridge to the outside when the penetrating element is inserted and for example used to penetrate a sealing element or septum. The longitudinal axis can be an axis which runs from one side of a septum to the opposite side. The penetrating element is seated or located on a sealing element which can be integral with or form part of the penetrating element but which preferably has no points or cutting edges. The cannula comprises at least one dispensing opening which should be on the outside of the cannula when the cannula is used to pierce a septum or access the inside of a vial or cartridge. Furthermore, the cannula comprises at least one and preferably two, three or more intake openings which are located on the inside of a vial or cartridge when the cannula is properly inserted. The at least one dispensing opening and the at least one intake opening are connected so as to allow a fluid path or the transportation of a substance from the at least one intake opening to the at least one dispensing opening.

The fluid path can be located inside the penetrating element and/or inside the sealing element and can have the shape of a cylindrical tube or opening or of a conical tube or opening, preferably widening from the inside of the cartridge to the outside of the cartridge when the cannula is inserted. The penetrating element preferably extends in the longitudinal direction beyond the at least one intake opening, i.e. when the cannula is inserted into a cartridge, the innermost part of the cannula is the penetrating element and preferably its tip, wherein in the longitudinal direction pointing towards the outer side of the inserted cannula, the at least one intake opening is located at a distance from the tip, i.e. there is no intake opening directly at the tip. At least one of the sharp or cutting edges of the penetrating element can at least partially overlap in the longitudinal direction with at least one intake opening while being offset in the circumferential direction, or is provided in the longitudinal direction in an area in which at least one intake opening is also provided. For example, when considering the penetrating element in a circumferential direction around the longitudinal axis in an area in which at least one intake opening is provided, at least one cutting or sharp element or edge is preferably provided at a longitudinal length corresponding to at least a part of the intake opening.

The at least one or each of the intake openings can be located within one or more axial passages extending e.g. from the tip of the penetrating element or from a point spaced axially inwardly from the tip and rearwardly through or along the cannula. The axial passages can be provided in the circumferential direction on the cannula and can e.g. be formed between respective adjacent cutting edges.

The penetrating element can be conical or can have the shape of a (right) circular cone, wherein a tip or piercing point is advantageously formed at a front end. The pentrating element can have outer or cutting edges being e.g. on a virtual surface (or enveloping surface) of a circular cone, i.e. if the edges were rotated around the longitudinal axis, they will move along the surface of a circular cone.

The penetrating element can be formed to have for example two, three, four or more segments in the circumferential direction, wherein each of the segments can be a segment of a cone or of the outside of a cone, wherein a cutting edge can be formed at the contact or connection area between two adjacent segments.

The shape of the penetrating element preferably consists of three abutting circular cone sections. The abutting circular cones result in a total of three sharp cutting edges at the tip of the cannula. The tip of the cannula can thus be geometrically divided into three segments. A core bore hole in the centre of the needle base can breach the side walls of the needle tip at three openings.

The openings are preferably formed as elongated holes. In terms of design, the openings can be arranged so as to be superimposed across zones. This means that after penetration, the needle opening comes to rest both inside and outside with respect to the septum.

Since the opening is not at the end, as with a steel needle, bubbles which may be adhered to the lower edge of the septum are easier to vent. The air bubbles can reach and enter the needle opening directly, without being obstructed. In a steel inventive needle, by contrast, the air bubbles have to surmount the distance (which is an obstacle) between the needle opening and the lower edge of the septum. Since the inventive needle tip protrudes further in the neck of the ampoule, the residual volume of air is smaller in this zone, which leads to improved occlusion recognition, since the ampoule pressure increases more rapidly when compressed. In order to exhibit better cutting properties, the penetrating element can be fitted with a tip which is for example made of metal. This can be achieved in an injection-moulding process using an insert part. Subsequently grinding or reshaping the cutting zone may also be considered.

In the cannula in accordance with the invention, the opening offset form the cannula tip comes to rest over a large longitudinal region, hence the opening lies both in the septum and outside the septum. This enables air (bubbles) to flow off more effectively. For reasons of manufacture, the cannula or needle is advantageously always centric and at right angles to the needle base.

When manufactured in an injection-moulding process, the individual segments can be shaped precisely using corresponding mould inserts or slides. Separating the segments has the advantage of forming sharp edges (cutting lines). During the filling process for the plastic, subsequently accumulating air can be removed through so-called venting channels. The injection pressure for shaping the thin cutting lines is therefore not reduced too much, which improves precision in shaping the cutting lines. This also enables burn marks caused by the injection process to be avoided. The conical circles in the region of the needle hole provide the tip of the cannula with an optimum breach which can be varied using the core of the mould.

The penetrating element can be formed from a single material, such as a plastic, or from a combination of several materials and can for example have a tip made of metal which is used as the piercing point or tip to be inserted or connected for example to plastics forming the rest of the penetrating element.

A sealing element, which is advantageously connected to the penetrating element, is preferably connected at the bottom end of the penetrating element opposite the tip or piercing end of the penetrating element and can be disc-shaped or saucer-shaped and/or can have a cylindrical or conical shape, in particular a frusto-conical shape which substantially continues the conical shape formed by the penetrating element or its cutting edges. A cylindrical cone can fulfil the function of forming a seal. The septum is more significantly displaced by the cone at the beginning of the penetration region. Once the needle has completely penetrated the septum, the enveloping force is even higher at this point. Due to the increased biasing force, the cannula to the septum has a better fit. It tolerates a greater deviation in centring. Leakage is reduced.

The at least one intake opening can have the shape of a round, elongated, oblong or extended hole and is preferably not provided at the penetrating or piercing end or tip of the penetrating element. If more than one intake opening is provided, the intake openings can be distributed in the circumferential direction, preferably equidistant around the above-defined longitudinal axis, and/or can be offset with respect to each other in the longitudinal direction. It is possible for the at least one intake opening to be provided within the sealing element and/or within the penetrating element.

Preferably, the cannula has an extension in the longitudinal direction of 6 mm or less.

The cannula can comprise a surface or contact area which provides a receiving part for a sealing element, such as an O-ring, as described below.

The sealing element can be connected to or provided with a needle base which is for example an assembly part and is preferably provided at the bottom end of the sealing element, opposite the end at which the sealing element is connected to the penetrating element. The cannula can be fitted with a needle base as a joining part for various functions. The needle base can be ideally centred, depending on its shape, with respect to another part. The multiple functionality of this part allows it for example to accommodate an O-ring for sealing a valve. It can also be configured such that it can be joined to another component by latching, etc. Injection-moulding considerations highlight another advantage of the needle base, since the accumulation of material has a positive effect during injections, due to the continuous reduction in filling compound towards the tip.

The needle base, the penetrating element and the sealing element can be formed from a single material, such as a plastic, or from a combination of several different materials.

In accordance with another aspect, the present invention relates to an adaptor for an ambulatory infusion pump which comprises and/or uses a cannula as described above.

The cannula described above provides one or more of the following advantages. It can be made entirely from plastic alone, hence no nickel components are used in the needle, which may cause allergic reactions. It is possible to penetrate various different sorts of vials or cartridges. The basic concept for penetrating or accessing the interior of a cartridge or vial is to cut a sealing element, such as a septum, instead of piercing or punching it, as is the case with known steel needles.

The cannula is preferably designed to have a shape and/or form such that when it is partially or fully inserted at an access site such as a septum, at least one of the intake openings is at least partially inside the cartridge and preferably extends from the inner surface or even from within the sealing element or septum into the cartridge. Thus, any gaseous substance or air contained within the cartridge can easily be discharged, and preferably no air remains in the cartridge after the cannula has been inserted, which provides an advantage for accurately dispensing the substance contained within the cartridge or for immediately detecting an occlusion.

Since no separate needle has to be introduced, as is the case when inserting the steel needle into a plastic adaptor as in the prior art, the needle can be formed directly on or even as part of the cannula or penetrating element.

The cannula is preferably made of a material based on synthetic or mineral substances which preferably do not contain nickel.

The cannula can have an inner diameter which is preferably in the range of 0.1 mm to 5 mm, such as the common inner diameter of 1.0 mm.

The cannula described above can be produced using injection die casting or injecting die moulding. Preferably, the cannula and the elements or components formed parts of the cannula or being connected to it, such as the penetrating element, the sealing element, and a base element, can be produced in a single step which can be performed in a single-component injection die casting machine in a single shot, thus, eliminating the additional steps of assembling or connecting individual components. It is also possible to manufacture the cannula using a two-component injection die casting machine.

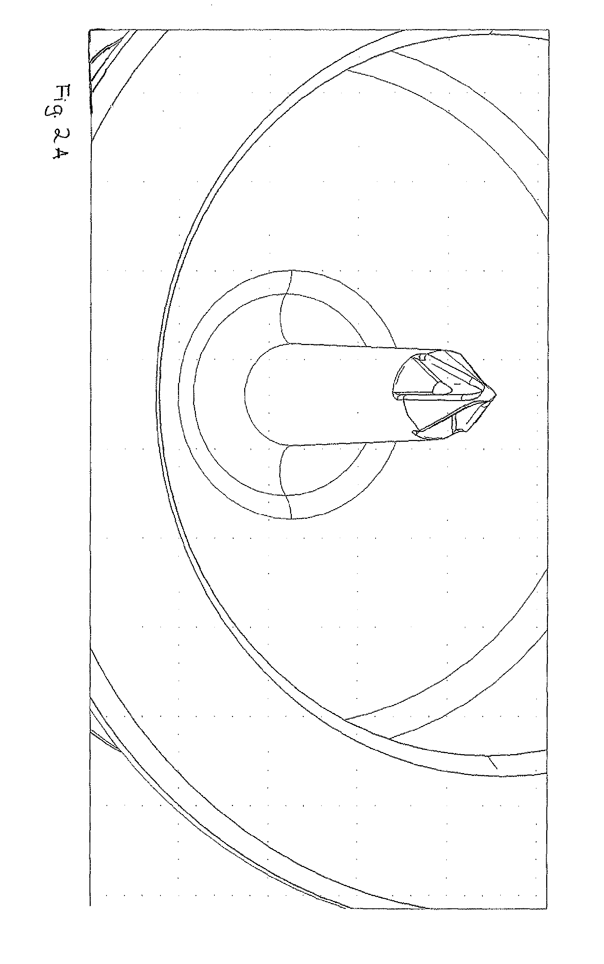

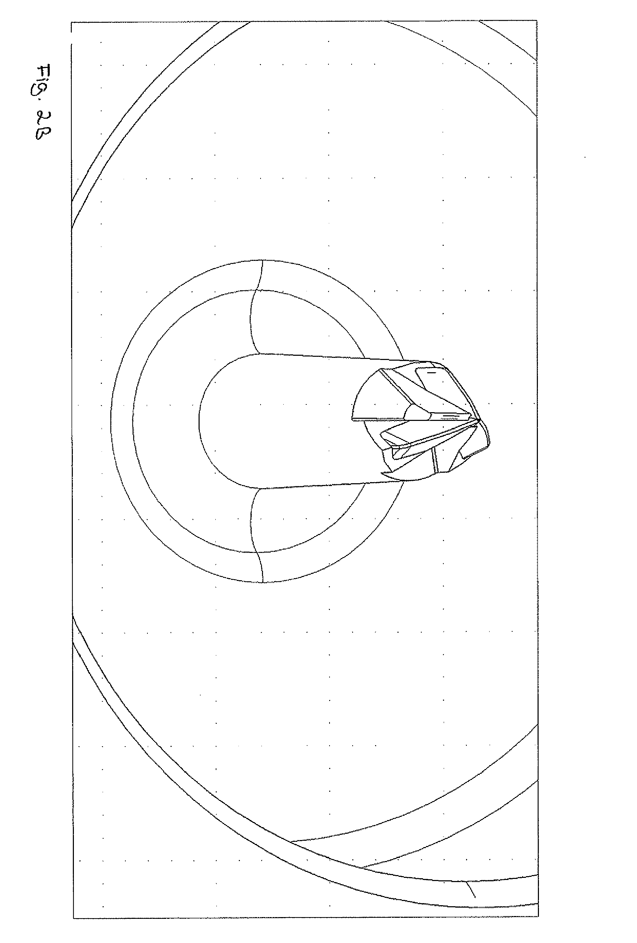

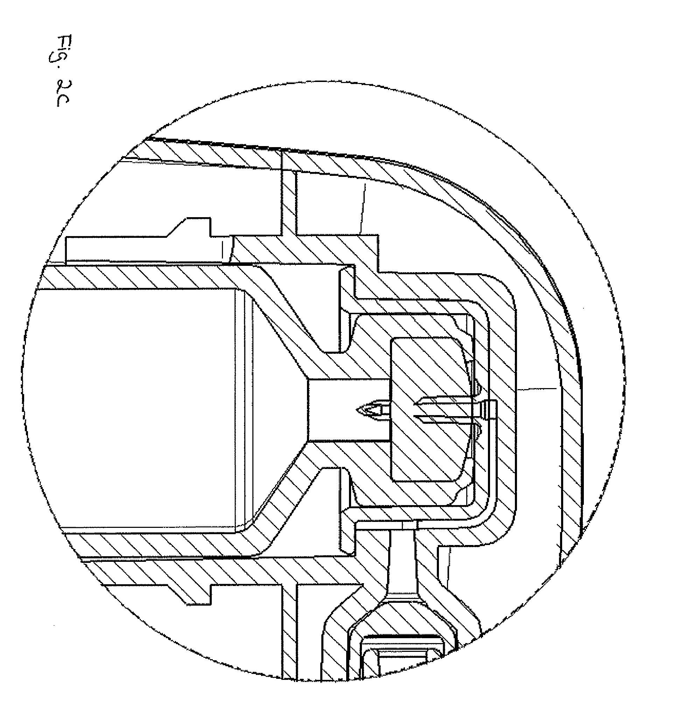

In a first embodiment of manufacturing the cannula, as shown in Fig. 2C, the fluid-guiding bore in the cannula is formed by a relatively long core which is supported on the opposite side on three segments. The contours which enable the penetration of the septum of the ampoule are shaped in the counter-mould, wherein the septum is penetrated centrically, which reduces the danger of the cannula running off centre as it penetrates through the septum and therefore also the danger of fluid leakage at this point. Embodiments 1a and 1b shown in Figs. 2A and 2B differ mainly in their geometry which generates the penetration of the septum of the ampoule.

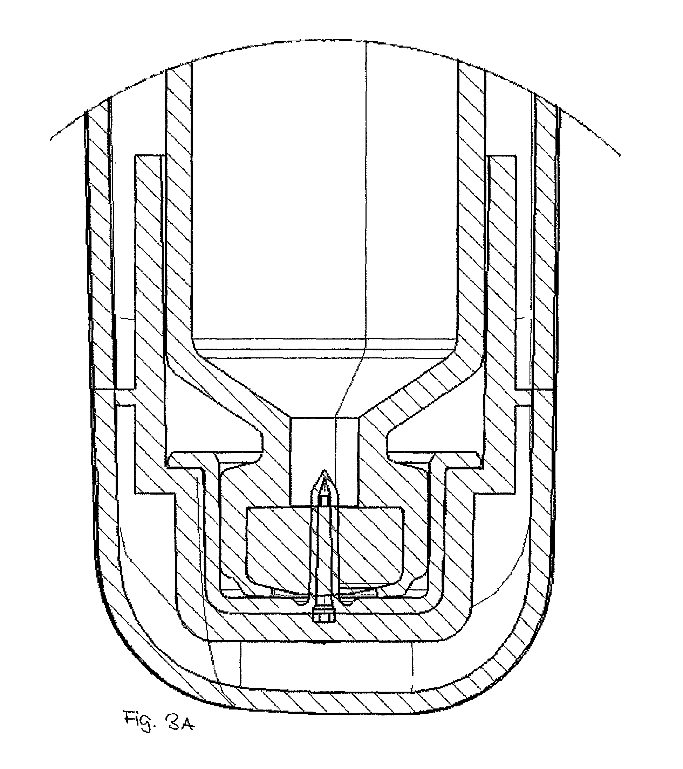

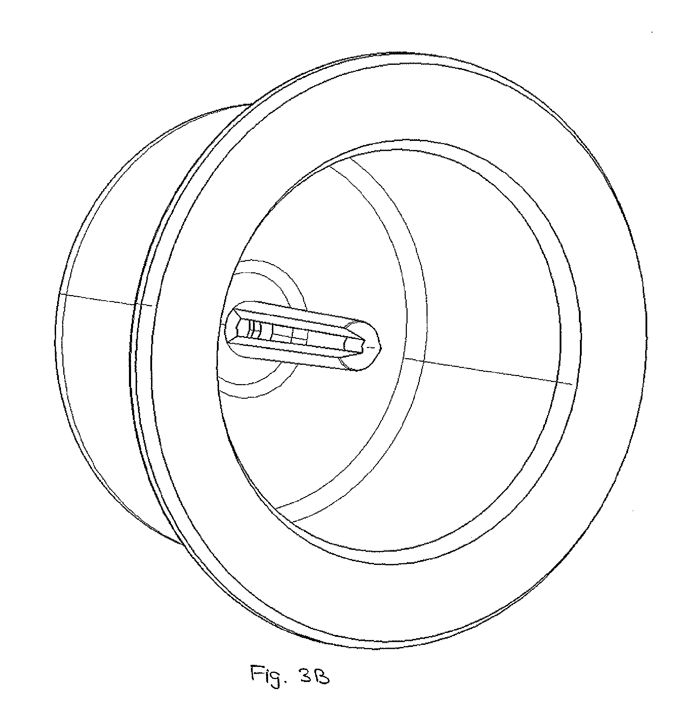

In the second embodiment shown in Fig. 3, the fluid-guiding bore is only formed by a relatively short core, the stability of which is given. The likewise fluid-guiding grooves on the outer side of the cannula are modelled in the injection mould in the counter-mould of the injection mould. The injecting tool is more robust as compared to the first embodiment, due to the lack of a long, thin core of the tool. The septum of the ampoule is again penetrated centrically.

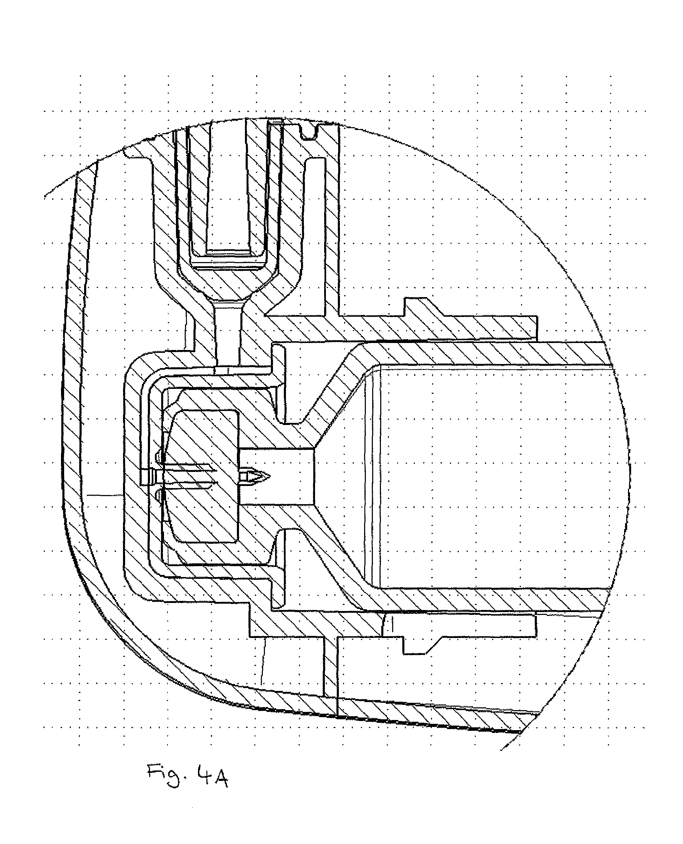

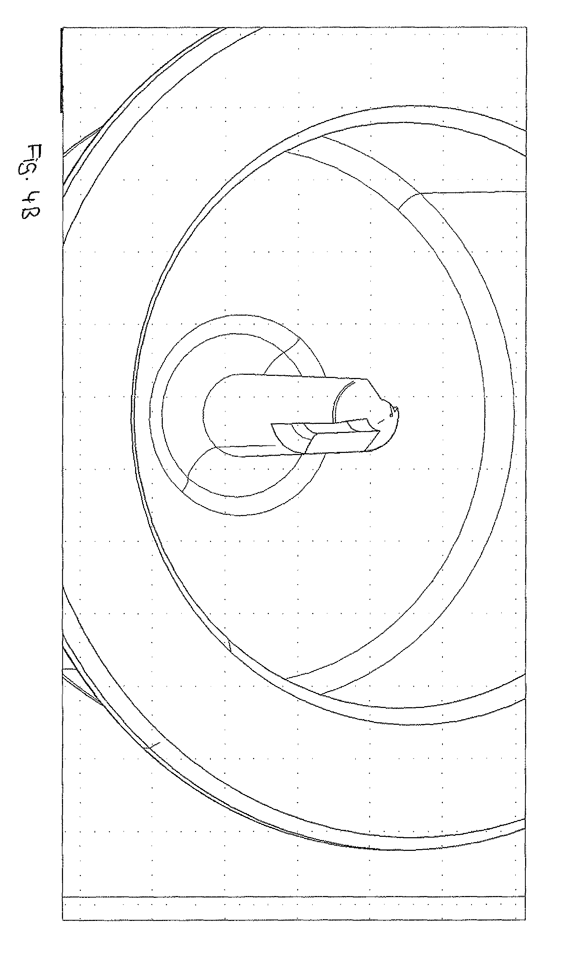

The third embodiment shown in Fig. 4 combines embodiments 1 and 2, i.e. the fluid-guiding bore is embodied to be longer than in embodiment 2, but not as long as in embodiment 1. This is intended to ensure that the septum of the ampoule envelops the cannula over a sufficient area and that no fluid leakage arises. The septum is also penetrated centrically in this embodiment.

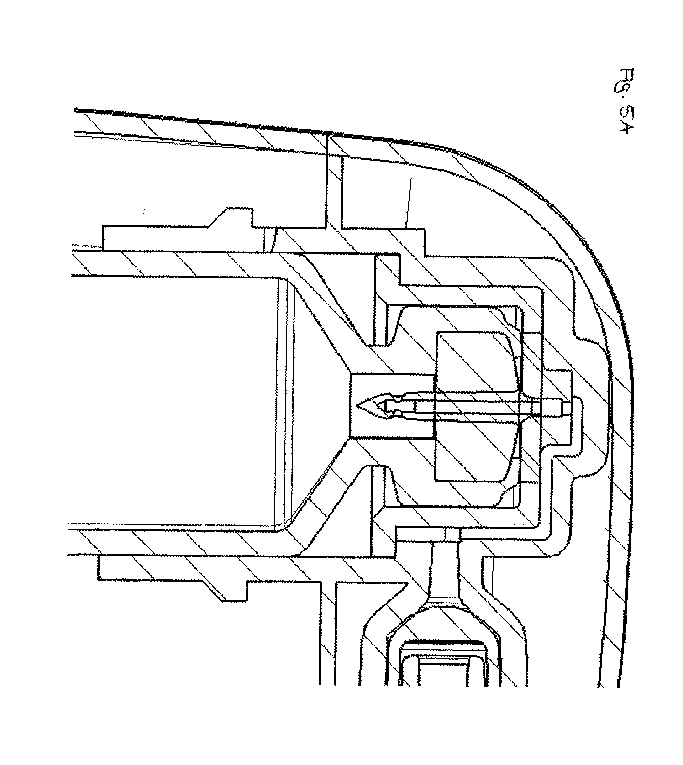

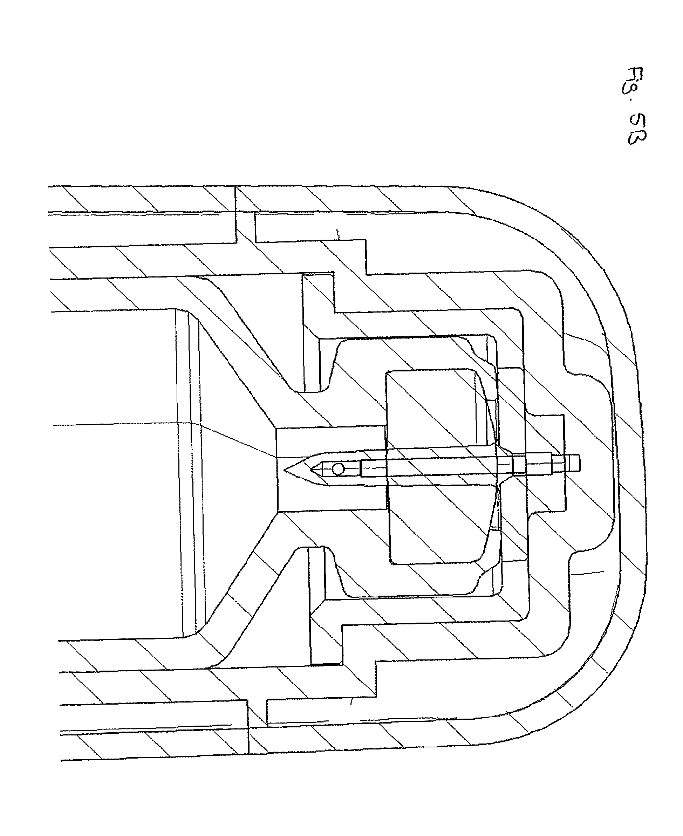

In Embodiment 4 shown in Fig. 5, the fluid-guiding bore is again formed by a long core, which can barely be supported in the injecting tool. Two bores running transversely are formed in the counter-mould of the tool and enable the fluid connection between the interior of the ampoule and the long, fluid-guiding bore. The septum is penetrated centrically.

In Embodiment 5 (not illustrated), the fluid-guiding bore is again formed by a long core, which is supported radially and circumferentially in the counter-mould to the injecting tool. The needle tip is also formed in the counter-mould, in an analogous way to a whetted steel cannula. The septum is probably not penetrated centrically.

The range of possible applications for the cannula can include one or more of the following: an interface between an adaptor and an ampoule; an interface between a catheter and the head of an infusion set; an interface between a catheter and an adaptor; an interface between two catheters; an interface between a filling aid and a vial, ampoule or the like; an interface between the head of an infusion set and a body; an interface for a fluid path within one or more modules; an interface for a pouch, wherein the film of the pouch is penetrated; an inversion, for example of a cannula on an ampoule; pre-punched or slit septa; barbs on a cannula; a positioning aid for soft cannulae, such as for example a guiding needle; glucose measuring devices; a general connecting method in medicinal technology, biotechnology, laboratory technology, pharmaceutics and/or diagnostics; bi-channel, bi-lumen solutions, in particular for a siphoning or transfusing aid; sharp, metal-coated (galvanised, PVD, etc.) tips; materials for injection-moulded processes (for example metal injection moulding, ceramics, possibly glass); possibly semi-permeable batches; the use of multi-lumen catheters.

In accordance with another aspect, the present invention relates to a valve which can be included in or used with a cannula, such as for example a cannula of the type described above or which can be used independent of such a cannula. Preferably, the valve can be used for an ampoule or a cartridge and/or pump.

The present invention relates in particular to a device for preventing a free catheter flow (free-flow prevention) and specifically to a cannula or valve element which is formed such that a free flow of a fluid through the cannula due to a hydrostatic pressure of the fluid being channelled in the catheter is prevented by the valve. The invention also relates to a system comprising such a catheter and an administering device, in particular an infusion pump.

Infusion systems store the drug to be administered in a container, usually a cartridge or an ampoule, in which a carrier fluid with the drug dissolved in it - referred to in the following simply as the drug fluid - is situated between a movable stopper and a container outlet. The rear end of a catheter is connected to the container outlet. A cannula or needle, which is for example connected via a catheter which is turn for example connected via a Luer connection to the cartridge to another needle which is introduced into a human or animal body in order to administer the drug fluid, is placed on the front end of the catheter and in most cases remains there for an administering period which is often several days, wherein if the container with the drug fluid is situated at a greater height than the front end of the catheter and/or the needle being introduced into the body, there exists the danger, if the difference in height between the container and the front end of the catheter is large enough, of the container gradually emptying itself due to the force of the column of fluid.

In insulin therapy using portable infusion apparatus, for example pump apparatus, the catheters used can exhibit lengths of more than 1 m. If the apparatus, including the container, is arranged vertically above the user, for example at night or when showering, a hydrostatic ground pressure of about 0.1 bars is generated, if no other effect - for example friction losses, discharge effects or capillary action - are taken into account, in addition to the purely static pressure due to the inherent weight of the drug fluid, and if the density of water is assumed for the drug fluid.

WO 2009/003560 A1 discloses a catheter comprising a catheter wall and a flow region limited by the catheter wall, for connecting an administering device to an administering needle, and comprising at least one catheter portion at which at least one partial piece of an inner wall of the catheter abuts at least one other partial piece of an inner wall of the catheter, in order to block or obstruct the flow of a medium through the catheter, wherein the flow is opened when the medium exhibits a pressure above a predetermined blocking pressure.

The valve of the invention may be used in combination with an insulin pump and can preferably fulfil the same function as a known valve, such as for example the valve formed by the catheter described in WO 2009/003560 A1. The valve of the invention can be realised using standard components, such as an O-ring and a standard spring. The valve of the invention is preferably included in an adaptor and/or cannula such as has been described above.

Preferably, the valve comprises or consists of a valve body, such as e.g. a cylindrical, conical, spherical or disc-shaped element, and a resilient or elastic or spring element which acts on the valve body and preferably exhibits a force on the valve body, such that the valve body is moved or pressed into a position in which the valve body comes into contact with a sealing element such as an O-ring, in order to stop or block the transportation of a substance or fluid. The valve body is preferably situated in the transporting or fluid channel for a substance to be dispensed which can be pumped or transported out of a cartridge. When the valve body is not or not fully in contact with the sealing element or O-ring, the substance or fluid to be delivered from the cartridge can flow through the opening formed by the valve body being pressed or moved away from the sealing element.

The spring element and/or the valve body are preferably designed such that a pressure of at least 0.1 bar or 0.2 bar or 0.3 bar is required to open the valve, i.e. is to move the valve body, which is being pressed by the spring element against the sealing element, in a direction away from the sealing element, against the force of the spring element. If the pressure of the substance or fluid acting on the valve or the valve body drops below the above-mentioned limit, for example below 0.2 bar, the spring element urges the valve body in a direction towards the sealing element, such that the valve body is pressed against the sealing element and blocks any further transportation of a fluid or substance.

Thus, a minimum pressure of for example 0.2 bar for delivering or transporting a substance from a cartridge is required in order to prevent a free flow of the fluid from the cartridge due to hydrostatic pressure.

The valve and in particular the valve body and the sealing element or O-ring is preferably formed from a plastic or an elastic material and preferably does not contain any nickel. The spring element can be any standard spring element and can be formed from plastic or any metal or alloy preferably not containing nickel.

The valve body, which is positioned on or in contact with the sealing element when the valve is closed, preferably has a direction of contact or pressure on the sealing element or O-ring which forms an angle of 60° with the direction of pressure caused by the spring element on the valve body.

The invention is described below with reference to embodiments illustrated by the attached figures, which show:
- Figure 1: a perspective view of a cannula in accordance with an embodiment of the invention;
- Figure 1A: a top view onto the cannula shown in Figure 1;
- Figure 1 B: a cross-sectional view along line A-A in Figure 1A;
- Figure 1C: a side view onto the cannula shown in Figure 1;
- Figure 1D: a cross-sectional view along line B-B in Figure 1C:
- Figures 2A to 2C: a penetrating element of the cannula formed in accordance with a first embodiment of manufacturing the cannula;
- Figures 3A and 3B: a penetrating element in accordance with a second embodiment of manufacturing the cannula;
- Figures 4A and 4B: a penetrating element in accordance with a third embodiment of manufacturing the cannula;
- Figures 5A and 5B: a penetrating element in accordance with a fourth embodiment of manufacturing the cannula;

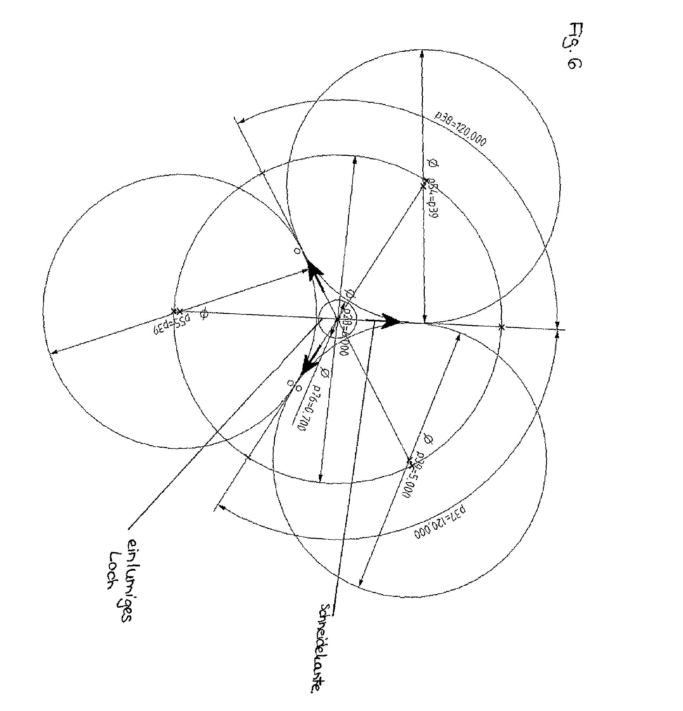

- Figure 6: the principle design of the tip of the penetrating element;
- Figure 7: a cross-sectional view of a cartridge accommodated within an infusion pump which is connected to the cannula in accordance with the invention;
- Figure 8: an enlarged view of the cannula of Figure 7;
- Figure 9: a cross-sectional view of another embodiment of a cannula which is inserted into a septum of a cartridge; and
- Figure 10: a steel cannula which is penetrated through a septum of a cartridge.

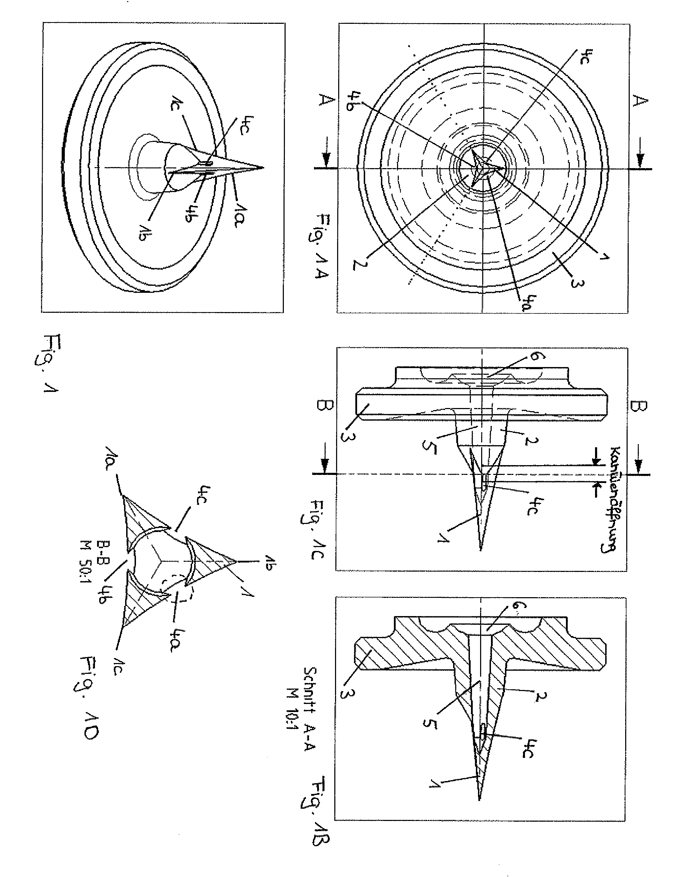

Figure 1 shows a perspective view of a cannula in accordance with a first embodiment, the top of which comprises a penetrating element 1 in which three intake openings 4a, 4b and 4c are included. The penetrating element 1 is connected to a sealing element 2 which is in turn connected to a base element 3. The penetrating element 1 is formed by three segments each having the form of a segment of a cone. The three segments form sharp cutting edges 1a, 1b and 1c in their respective connection areas.

As shown in Figure 1A, the sealing element 2 and the base element 3 are rotationally symmetrical about the longitudinal axis running through the point formed by the tip of the penetrating element 1.

As can be seen from Figures 1B and 1C, each of the intake openings 4a to 4c connects the exterior of the cannula or penetrating element 1 to a through-hole 5 formed within the penetrating element 1, the sealing element 2 and the base element 3. The through-hole 5 forms at its end opposite the position of the intake openings 4a to 4c a dispensing opening 6, such that the intake openings 4a to 4c and the dispensing opening 6 are in fluid communication via the through-hole 5. The through-hole 5 and the dispensing opening 6 are rotationally symmetrical about the longitudinal axis of the cannula.

The cutting edges 1a to 1c of the penetrating element 1 extend in a longitudinal direction from the tip of the penetrating element 1, shown on the right-hand side in Figure 1C, past the intake openings 4a to 4c to the sealing element 2.

The intake openings 4a to 4c are formed in channels extending in the longitudinal direction and formed between respective cutting edges 1a, 1b and 1c.

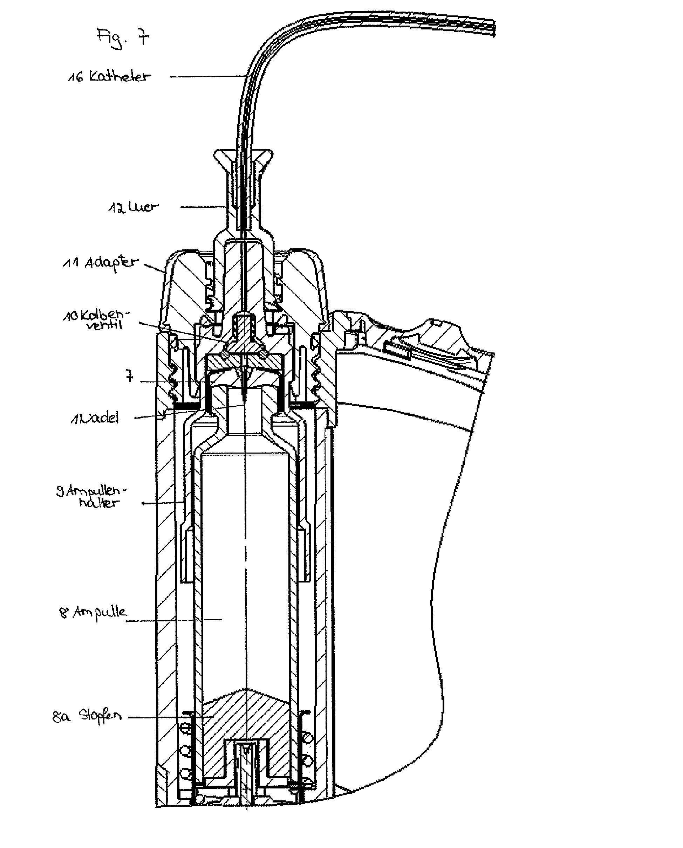

If the cannula is placed, via the tip of the penetrating element 1, on top of a septum 7 of a cartridge 8, in order to pierce or cut the septum 7 and so to provide a fluid path from the inside of the cartridge 8 to a catheter 9, as illustrated in Figures 7 to 9, then inserting the cannula into the septum 7 of the cartridge 8 can be divided into two phases:

In a first phase, the penetrating phase, the tip of the penetrating element 1 cuts open the septum 7. In the second phase, the cylindrical cone of the sealing element 2 is slid into the septum 7, in order to form a seal. The three longitudinal cutting edges 1a to 1c along the penetrating element 1 (the tip of the cannula) fulfil the function of cutting open the septum 7. The length of penetration depends on the diameter of the sealing element 2 and on the acuity angle of the needle. The needle opening can be positioned at any point in terms of its design.

The total length is less than 6 mm, i.e. only half the length of conventional steel cannula. The total height of an adaptor or of the pump can therefore be reduced by a few millimetres. The space thus saved can for example be used to install a valve without increasing the total length of the apparatus (pump).

The cannula can be provided with numerous functions. It can for example comprise shape geometries for accommodating standard parts or latching connections for coupling to another part. As a two-component element, it can for example be provided with a seal or can exhibit a number of openings which fulfil various functions.

The interior of the cartridge 8 located between the plug 8a and the septum 7 contains a substance or fluid to be dispensed. The cartridge 8 is held by a cartridge holder 9 of an infusion pump. An adaptor 11 is positioned at the dispensing opening 6 of the cartridge 8 and holds in its interior the above-described cannula together with the valve 10. The outer end of the cartridge holder 9 comprises a Luer connection, to which a catheter 16 can be connected. An infusion set can be attached at the end of the catheter 16 opposite the end which the Luer connection 12.

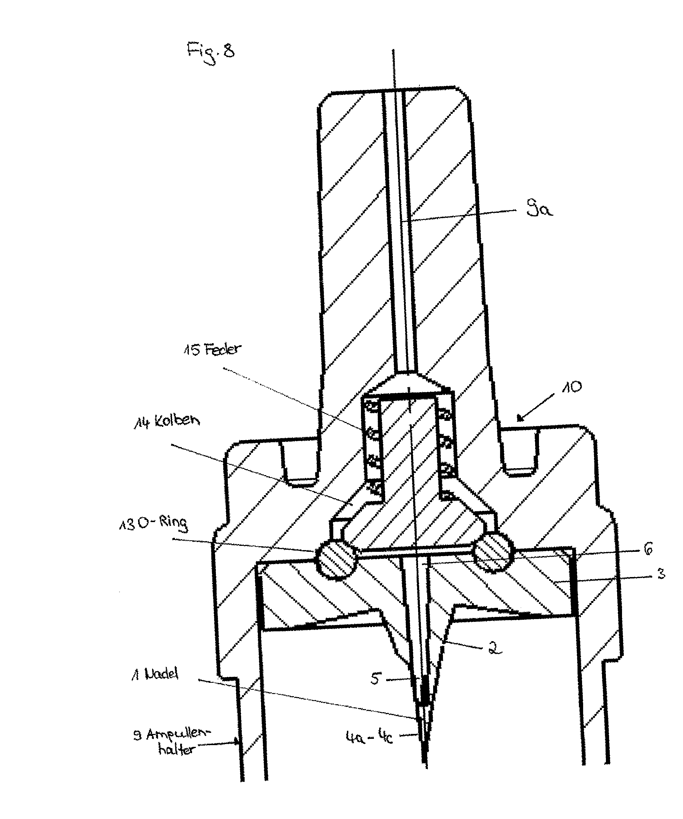

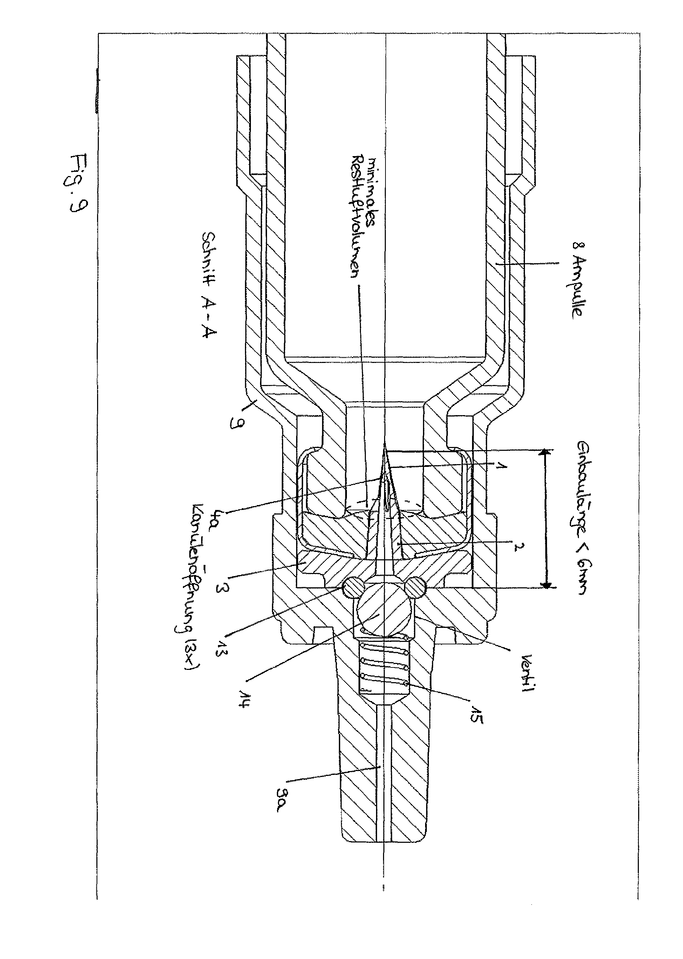

Figure 8 shows a cross-sectional view of the cartridge holder 9 which holds the above-described cannula 16. The tip of the penetrating element 1 points towards the cartridge 8. At the opposite end of the penetrating element 1, the cannula comprises a seat or holding portion for an O-ring 13 which functions as a sealing element when acting together with the valve body 14. The valve body 14 is biased towards the O-ring 13 by a spring 15, thus blocking the fluid path from the interior of the cartridge 8 through the intake openings 4a to 4c the through-hole 5 and the dispensing opening 6, along the bottom face of the valve body 14 past the outer side of the O-ring 13 and
in the longitudinal direction of the valve body 14, into the through-hole 9a of the cartridge holder 9 and on through the catheter 16 to an infusion set (not shown). Fig. 9 shows a further embodiment of a cannula having a ball-shaped or spherical valve body 14 being pressed against the sealing element 13 by the spring 15.

If a pressure of more than 0.2 bar acts on the valve body 14, generated for example by the infusion pump acting on the plug 8a, the valve body 14 is moved against the bias of the spring 15, such that the valve is opened and fluid can be dispensed.

## Claims

1. Cannula for piercing a septum (7) of a vial or cartridge (8) comprising a penetrating element (1), at least one dispensing opening (6) of the cannula being in fluid connection with at least one intake opening (4a, 4b, 4c), wherein the penetrating element (1) comprises at least one cutting edge (1a, 1b, 1c) extending in a longitudinal direction of the penetrating element (1) to at least partially overlap in the longitudinal direction with the at least one intake opening (4a, 4b, 4c).

2. Cannula according to claim 1, wherein the at least one intake opening (4a, 4b, 4c) is/are offset from the at least one cutting edge (1a, 1b, 1c) in the circumferential direction of the cannula.

3. Cannula according to claim 1 or 2, wherein the penetrating element (1) is conical or has the shape or a bounding surface or enveloping surface of a circular cone or right circular cone.

4. Cannula according to one of the preceding claims, wherein the penetrating element (1) is formed of at least three cone segments or segments of the outer side of a cone.

5. Cannula according to one of the preceding claims, comprising at least two or three cutting edges (1a, 1b, 1c) located at the outside of the penetrating element (1).

6. Cannula according to one of the preceding claims, comprising at least two or three intake openings (4a-4c) which can be located in longitudinal passages of the cannula and/or between adjacent cutting edges (1a, 1b, 1c).

7. Cannula according to one of the preceding claims, wherein a tip of the penetrating element (1) is formed of metal.

8. Cannula according to one of the preceding claims comprising a sealing element (2) being connected to the penetrating element (1) at the side opposite to the tip or point of the penetrating element (1).

9. Cannula according to the preceding claim, wherein the sealing element (2) is conical or frusto-conical or cylindrical.

10. Cannula according to one of the preceding claims, wherein the at least one intake opening (4a-4c) has the shape of an elongated or long hole.

11. Cannula according to one of the preceding claims, wherein each of the at least one intake openings (4a-4c) is spaced apart in longitudinal direction from the tip or point of the penetrating element (1) by a predetermined distance.

12. Cannula according to one of the preceding claims being formed of a single material such as plastics or a plastic material.

13. Cannula according to one of the preceding claims having a longitudinal length of less than 7mm or less than 6mm.

14. Cannula according to one of the preceding claims comprising a receiving part for a sealing element or O-ring.

15. Cannula according to one of the preceding claims comprising a needle base (3) or assembly part preferably being disc-shaped.

16. Cannula according to one of the preceding claims comprising a valve having a spring (15), a valve body (14) being biased by the spring (15) in a direction to come into contact with a sealing element (13) to block a fluid path if the fluid pressure on valve body (14) acting against the force of spring (15) is less than a predetermined pressure.

17. Adapter for an ambulatory infusion pump comprising a cannula according to one of the preceding claims.

18. Cannula according to the preceding claim, wherein the predetermined pressure is 0.1 bar, 0.2 bar or 0.3 bar.

19. Method for manufacturing a cannula of one of claims 1 to 17 by injection die casting or injection die moulding, preferably in a single shot.
